Europäisches Patentamt

European Patent Office

Office européen des brevets

(11) Veröffentlichungsnummer: **0 146 572**
**B1**

(12) **EUROPÄISCHE PATENTSCHRIFT**

(45) Veröffentlichungstag der Patentschrift:
10.01.90

(51) Int. Cl.⁴: **C 12 N 15/00, C 12 P 21/02, C 12 N 1/20**

(21) Anmeldenummer: 84901999.7

(22) Anmeldetag: 23.05.84

(86) Internationale Anmeldenummer:
PCT/EP 84/00154

(87) Internationale Veröffentlichungsnummer:
WO 84/04755 (06.12.84 Gazette 84/28)

(54) **VERFAHREN ZUR STEUERUNG DER EXPRESSION VON KLONIERTEN GENEN.**

(30) Priorität: 27.05.83 DE 3319242

(43) Veröffentlichungstag der Anmeldung:
03.07.85 Patentblatt 85/27

(45) Bekanntmachung des Hinweises auf die Patenterteilung:
10.01.90 Patentblatt 90/2

(84) Benannte Vertragsstaaten:
AT BE CH DE FR GB LI NL SE

(56) Entgegenhaltungen:
EP-A- 0 041 767
US-A- 4 278 765
US-A- 4 374 927

Proc. Natl. Acad. Sci, USA, Vol. 80, 1983, H. DE DeBoer et al.:"The tac promoter: A functional hybrid derived from the trp and lac promoters", see pages 21-25, see the whole document
Mol. Gen. Genet. (1981) 183, p. 202-204

Die Akte enthält technische Angaben, die nach dem Eingang der Anmeldung eingereicht wurden und die nicht in dieser Patentschrift enthalten sind.

(73) Patentinhaber: BATTELLE DEVELOPMENT CORPORATION, 505 King Avenue, Columbus Ohio 43201 (US)

(72) Erfinder: HÄNGGI, Urs, Frankfurter Strasse 94, D-6238 Hofheim (DE)
Erfinder: MIESCHENDAHL, Martin, Burgstrasse 116, D-5060 Bergisch-Gladbach 1 (DE)
Erfinder: PETRI, Thomas, Bad Sodener Strasse 5, D-6231 Sulzbach (DE)

(74) Vertreter: Sartorius, Peter, Dipl.-Ing., Battelle-Institut e.V. Abteilung Patente Am Römerhof 35, D-6000 Frankfurt am Main 90 (DE)

## Beschreibung

Die Erfindung betrifft ein Verfahren zur Steuerung der Expression von in einem Vektor inserierten Genen in prokaryotischen Mikroorganismen durch Verwendung eines Vektors, der einen Expressionskomplex enthält, wobei der den Vektor enthaltende Mikroorganismus in einem Chromosom oder in einem mit dem Vektor verträglichen Plasmid oder Episom ein Kontrollelement enthält und die Steuerung der Expression durch Änderung der Konzentration von Aminosäuren durchgeführt wird.

Eines der Ziele der industriellen Gentechnik ist es, die Gen-Expression zu steigern. Dieses Ziel wird mit herkömmlichen Vektoren nur bedingt erreicht, da hohe Expressionsraten sich negativ auf das Zellwachstum auswirken. Es ist daher versucht worden, Vektoren zu konstruieren, bei denen die Expressionsrate durch externe Eingriffe reguliert werden kann. Dadurch soll erreicht werden, daß während der Wachstumsphase der Mikroorganismen kein oder nur wenig Produkt gemacht wird, so daß sich der negative Einfluß der Fremdprodukte wenig oder gar nicht auswirkt. Die Produktion soll dann erst am Ende der Wachstumsphase induziert werden.

Die externe Steuerung kann z.B. über Veränderung der Inkubationstemperatur erfolgen. So werden in sognannten «Runaway»-Plasmiden bei der Erhöhung der Inkubationstemperatur auf 41 °C die Anzahl der Gen-Kopien stark erhöht, was zu einer wesentlichen Steigerung der Expressionsraten der in den Plasmiden klonierten Gene führt (M. Bittner et al. (1981) Gene 15, 319–329). In anderen temperaturgesteuerten Vektoren wird die Labilität von temperatursensitiven Repressor-Molekülen bei höheren Temperaturen ausgenutzt. Die Inaktivierung dieser Repressoren hat zur Folge, daß die Blockierung der Promotoren aufgehoben und daß die klonierten Gene voll exprimiert werden. Hier hat sich das λ-System hervorragend bewährt (E. Remaut et al. (1981) Gene 15 81–93).

Die Steuerung der Gen-Expression in regulierbaren Vektoren kann auch auf chemischem Weg erfolgen. Bei den sogenannten katabolisch regulierten Expressions-Vektoren werden nach der Wachstumsphase chemische Substanzen zum Medium gegeben, durch welche die Repressor-Moleküle inaktiviert werden. Damit wird ähnlich wie bei den temperatursensitiven Repressoren bei erhöhten Temperatur die Blockierung der Promotoren aufgehoben und die Expression der klonierten Gene erreicht. Typische Beispiele sind die lac-Vektoren, welche durch IPTG oder andere Galactoside induziert werden (U. Rüther et al. (1982) Nucl. Acids Res. 10, 5765). Die Steuerung der anabolisch regulierten Expressions-Vektoren basiert auf der Beobachtung, daß Zellen viele Bausteine sowohl selbst synthetisieren wie auch aus der Nährlösung aufnehmen können. Bei einem externen Angebot sind die Gene, die für die Synthese der Bausteine verantwortlich sind, abgeschaltet. Beim Absinken des externen Überschusses werden sie dereprimiert. In den Vektoren wird diese Steuerung so ausgenutzt, daß der Bausteinspiegel in der Nährlösung so lange hoch gehalten wird, bis die Produktionsphase erreicht ist. Durch die Depletion der Bausteine wird dann die Expression der klonierten Gene induziert. Beispiele sind Phosphatdepletionsensitive Vektoren (W. Boidol et al. (1982) Mol. Gen. Genet. 185, 510–512) oder Tryptophandepletion-sensitive Vektoren (H.A. Boer et al. (1983) Proc. Natl. Acad. Sci. USA 80, 21–25).

Verfahren der eingangs genannten Art zur Steuerung der Expression durch Änderung von Aminosäurekonzentrationen sind in der US-A 4 374 927 beschrieben.

Temperatur-steuerbare Expressions-Vektoren sind in der Regel nur in Versuchen im Laboratoriumsmaßstab problemlos. In der Anwendungstechnik in Fermentern von 5000 und mehr Litern bereiten sie Schwierigkeiten. So müssen z.B. Mikroorganismen mit Temperatur-sensitiven Expressions-Vektoren bei niedriger Temperatur angezogen und vermehrt werden. Dies hat zur Folge, daß die Produktionskosten wegen niedrigen Wachstumsraten und erhöhter Infektionsgefahr hoch sind. Zudem bereitet eine rasche Temperaturerhöhung in vielen Fermentertypen Schwierigkeiten. Bei den Depletions-sensitiven anabolischen Expressions-Vektoren muss die Konzentration der meist essentiellen Bausteine in dem Moment erniedrigt werden, in dem hohe Konzentrationen für maximale Expression erwünscht sind. Dieses Problem ist bei den Induktions-Vektoren nicht gegeben. Sie haben aber den Nachteil, daß meistens komplexe und teuere Chemikalien als Induktoren zur Nährlösung gegeben werden müssen. Während die Kosten für den Laboratoriums-Versuch tragbar sind, werden sie jedoch für die technische Anwendung prohibitiv.

Der Erfindung liegt daher die Aufgabe zugrunde, ein Hochleistung-Wirts-Vektor-System für die industrielle Großanlagen-Fermentation zu schaffen, mit dem

1. während der Wachstumsphase die Expression der klonierten Gene möglichst weitgehend verhindert wird, um negative Einflüsse auf das Zellwachstum auszuschließen;

2. in der Produktionsphase ein Maximum an Gen-Expression ermöglicht wird;

3. die Steuerung weder über Temperatursprung- noch über Depletions-Mechanismen erfolgt; und bei dem

4. die Induktoren preiswert sind.

Insbesondere liegt der Erfindung die Aufgabe zugrunde, ein Wirts-Vektor-System zu schaffen, bei dem die Expression der im Vektor klonierten, für ein oder mehrere für nützliche Produkte kodierende Gene unter der Kontrolle einer für sie fremden Promotor-Operator-Sequenz steht und durch das zugehörige Repressorprotein gesteuert wird. Das Gen für dieses Repressor-Protein soll sich gesondert davon auf dem Chromosom des Wirts oder in einem Plasmid oder in einem Episom im Wirt befinden. Das Plasmid oder Episom muß mit dem Vektor verträglich sein. Das Repressor-Gen steht seinerseits unter der Kontrolle einer fremden Steuerungskomponente, die ihrerseits durch ein Korepressor gesteuert wird, der mit dem Repressor einen aktiven Holorepressor bildet.

Diese Erfindung, wie sie in den Ansprüchen 1 bis 10 beschrieben ist, löst diese Aufgaben.

Erfindungsgemäß wurde das λ-System als Grundlage für einen neuen, induzierbaren Hochleistungsvektor genommen. Die Steuerung der biologischen Aktivität des Repressors erfolgt jedoch nicht wie bisher über Temperatursprung, sondern über seine Synthese unter der Regulation eines anabolisch wirkenden Kontrollelementes, spezifisch des Tryptophan-Promotors.

Die Erfindung wird in beiliegenden Zeichnungen näher beschrieben. Es zeigen:

Fig. 1A und 1B Konstruktionen des Vektorelementes;

Fig. 2 Konstruktion des Steuerelementes und

Fig. 3 Funktion des Wirts-Vektorsystems.

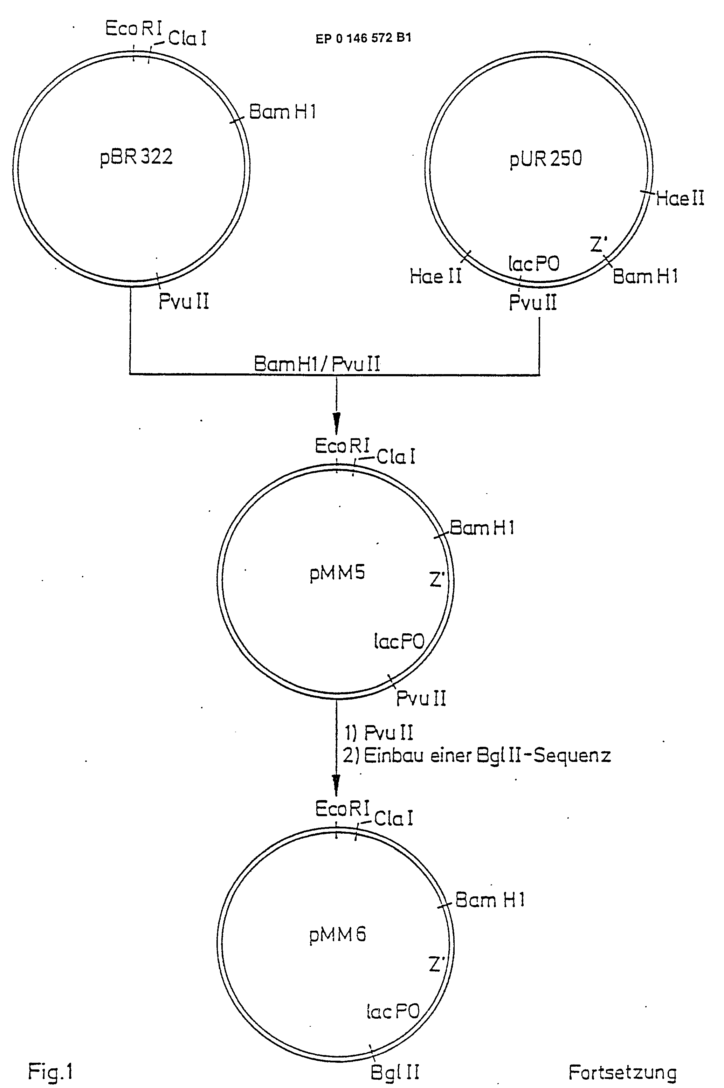

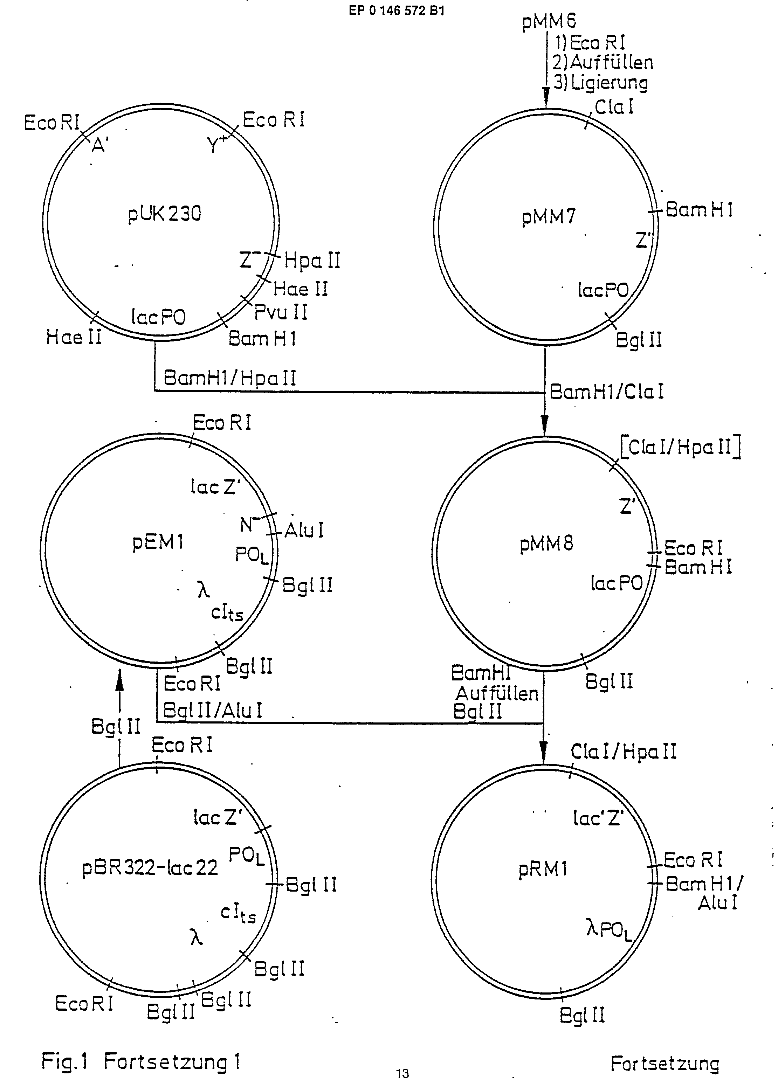

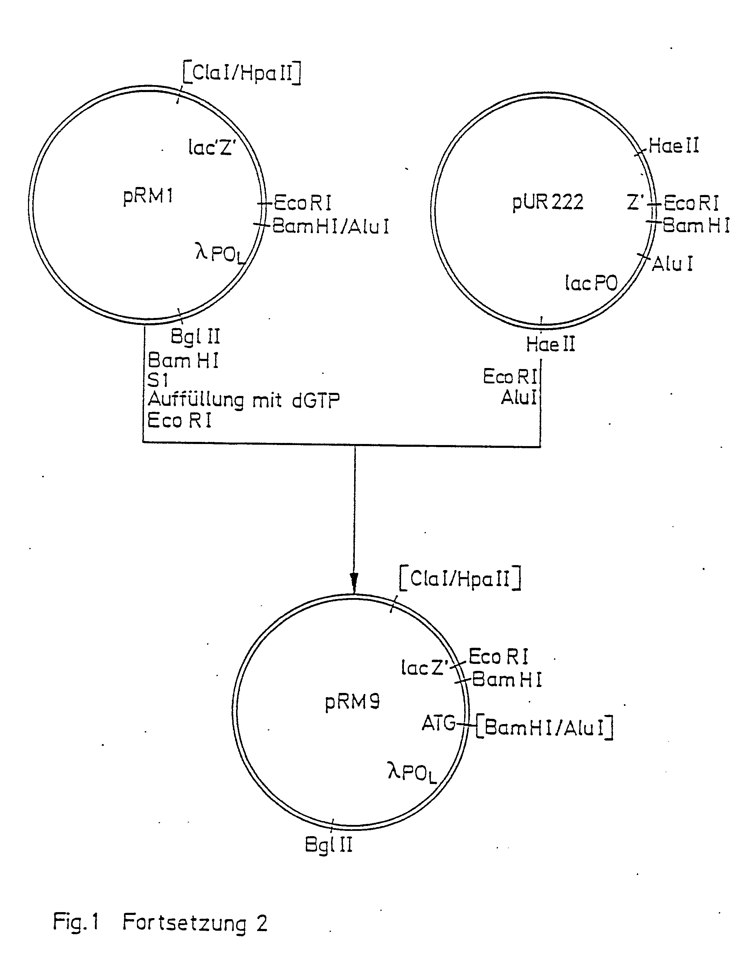

Das erfindungsgemäße regulierbare Plasmid wird gemäß Fig. 1 wie folgt hergestellt:

–pMM5: Das Pvull/BamH1-Fragment des Plasmides pUR250 (U. Rüther et al (1982) Nucl. Acids, Res. 10, 5765), das den lac-Promotor enthält, wird zwischen den Pvull- und BamH1-Spaltstellen in pBR322 (F. Bolivar et al., (1977) Gene 2, 95–113) kloniert.

–pMM6: Die Pvull-Site von pMM5 wird geöffnet und durch Einbau von Oligonukliden in eine Bglll-Spaltstellen umgewandelt.

–pMM7: Die später störende EcoRI-Spaltstelle wird durch Spaltung mit EcoRI, Auffüllen der überstehenden Enden mit DNS-Polymerase und «blunt end»-Ligierung zerstört.

–pMM8: Der in pMM7 fehlende Teil der β-Galaktosidase-Komplementations-Gen-Region wird als BamH1 Hpall-Fragment aus pUK230 (M. Koenen et al. (1982) EMBO. J. 1, 509–512) zwischen der BamH1- und Clal-Spaltstellen von pMM7 eingebaut.

–pEM1: Aus pBR322-lac22 (M. Mieschendahl et al., (1981) Mol. Gen. Genet. 183, 202–204) wird durch Bglll-Spaltung und Ligierung ein ca. 650 bp und ein ca. 60 bp Bglll-Fragment deletiert.

–pRM1: Aus PEM1 wird das Bglll/Alul-Fragment mit dem linken λ-Promotor zwischen die Alul-Sites am 5'-Ende des β-Glaktosidase-Gens und der Bglll-Site von pMM8 gesetzt. Das resultierende Plasmid PRM1 setzt sich zusammen aus dem λ-Promotor-Element und einem inaktiven α-Fragment des lacZ Gens.

–pRM9: Zwischen die EcoRI-Spaltstelle und die S1-behandelte BamHl-Spaltstelle von pRM1 wird ein EcoRl/Alul-Fragment von pUR222 (U. Rüther et al. (1981) Nucl. Acids Res. 9, 4087–4098) eingesetzt. Das resultierende Plasmid setzt sich zusammen aus dem linken λ-Promotor-element mit einer aktiven lac α-Region mit einem Polylinker aus pUR222.

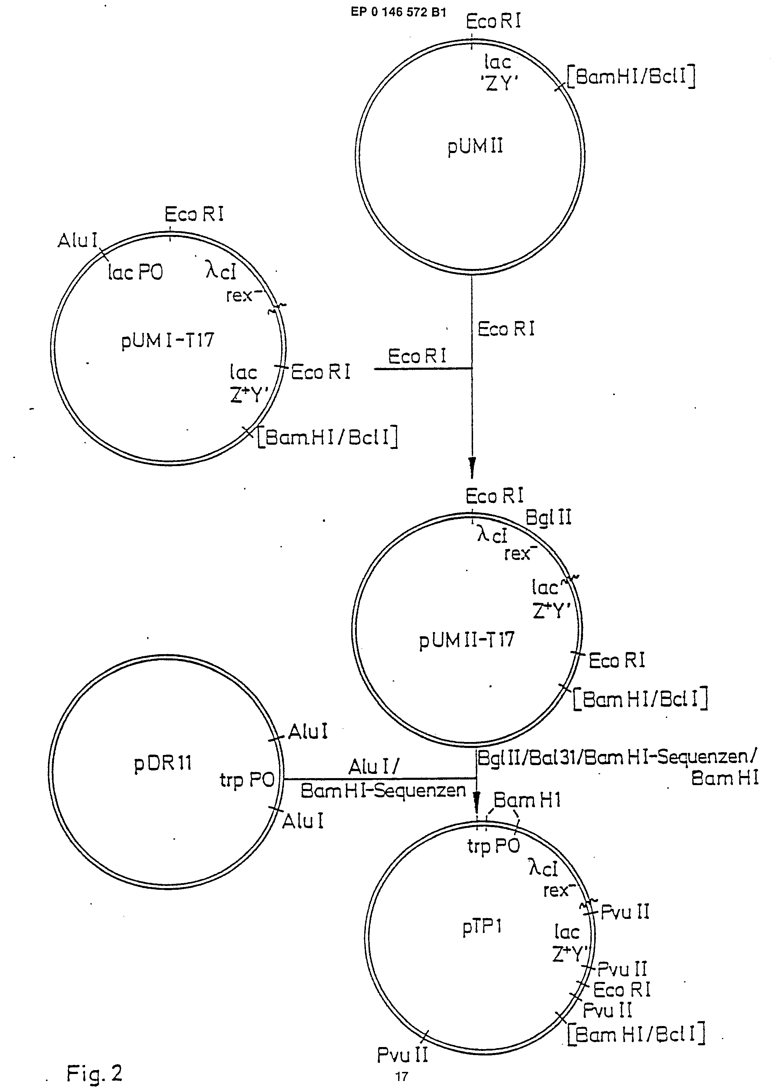

In Fig. 2 wird das Steuerungselement erläutert. Hierbei wird wie folgt vorgegangen:

–pUMII–T17: Das 3'-Ende des lac-Z-Gens von pUMII wird ergänzt durch die 5'-Ende des Gens aus einer λ rex-lacZ-Fusion durch Spaltung mit EcoRI und Insertion eines EcoRI-Fragmentes mit der λ rex-lacZ-Fusion von pUMI–T17 (M. Mieschendahl (1981) Dissertation, Universität Köln).

–pTP1: pUMII–T17 wire mit Bglll gespalten und mit Bal31 teilweise verkürzt. In das verkürzte Plasmid wird das Tryptophan-Regulationselement von PDR11 (D.R. Russell und G.N. Bennet (1982) Gene 17, 9–18) eingebaut.

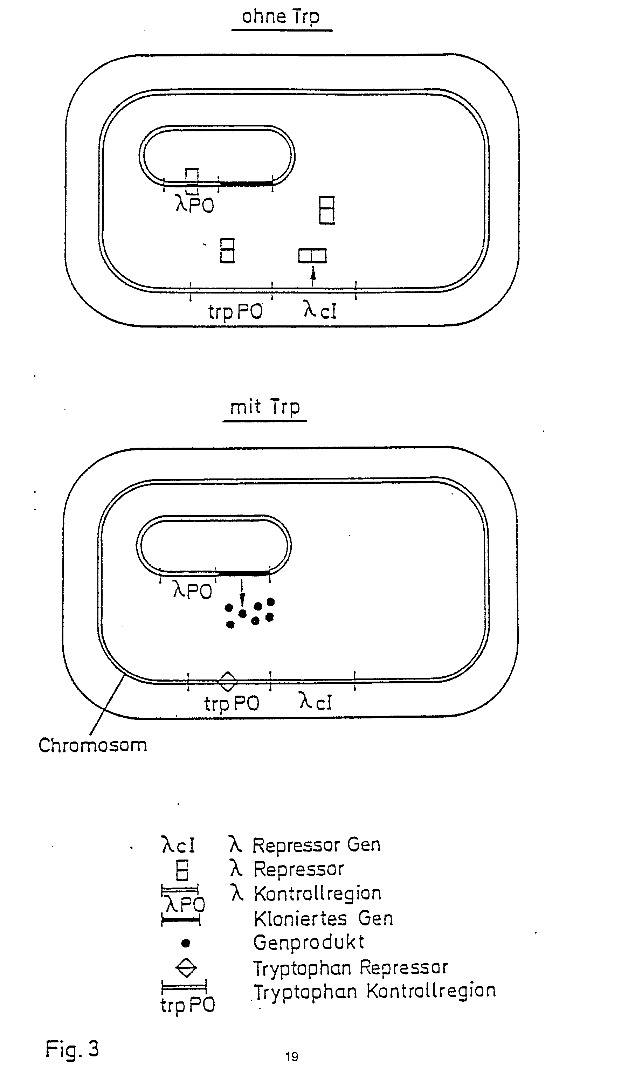

Im vorgeschlagenen Vektor-System wird gemäß Fig. 3 folgendes Steuerungsschema erreicht: In der Wachstumsphase wachsen die Mikroorganismen in einem billigen Standardmedium, in dem die Tryptophan-Konzentration niedrig ist. Da Tryptophan wesentlicher Bestandteil für die Zelle ist, sind die Tryptophan-synthetisierenden Gene aktiv. Das hinter dem Tryptophan-Regulationselement klonierte Repressor-Gen wird ebenfalls exprimiert, bindet an den λ-Promotor und blockiert die Expression der hinter den λ-Promotor klonierten Gene (= keine Expression). Vor dem Beginn der Produktionsphase wird das Wachstumsmedium mit Tryptonen, Peptonen o.a.m. oder Tryptophan supplementiert. Die Supplemente enthalten viel Tryptophan. Das Tryptophan reprimiert die intrazelluläre Tryptophan-Synthese und schaltet zugleich die Synthese des λ-Repressors unter der Tryptophan-Kontrolle ab. Wegen der fehlenden Neusynthese des Repressors wird nun der Promotor im Vektor dereprimiert und aktiviert. In der Folge werden die klonierten Gene maximal exprimiert.

Bei der Tryptophan-Steuerung wird erreicht, daß die Regulation durch Zugabe einer chemischen Substanz und nicht über Depletion- oder Temperatursprung-Mechanismen erfolgt. die Zugabe der regulierenden Substanz in Form von Peptiden oder Peptonen ist relativ preiswert und bietet zusätzlich den großen Vorteil, daß die Expression der klonierten Gene unter optimalen Nährstoffbedingungen erfolgen kann.

Die Erfindung wird in den nachfolgenden Beispielen detaillierter erläutert.

Beispiel 1
Isolierung von Plasmid DNS

Plasmid DNS wird in an sich bekannter Weise präpariert (U. Rüther et al. (1982), Proc. Natl. Acad. Sci. 79, 6852).

5 ml LB-amp Medium aus 10 g Trypton 5 g Yeast Extrakt, 5 g NaCl pro Liter doppelt destilliertem $H_2O$ (J. Miller (1972), Experiments in Molecular Genetics, Cold Spring Harbor Laboratory, New York), das 100 µg/ml Ampicillin enthält, wird mit einem plasmidhaltigen E. coli K12 Stamm infiziert und 16 Std bei 30 °C bebrütet. Die Übernachtkultur wird abzentrifugiert (Sorvall, SS34 Rotor, 5 Min 5000 UpM), der Niederschlag in 1 ml 25% Saccharose, 50 mM Tris-HCl pH 8.0 resuspendiert. Nach Zugabe von jeweils 0.1 ml Lysozym (20 mg/ml in 25% Saccharose, 50 mM Tris-HCl pH 8.0) und EDTA (0.5 M pH 8.0) in Abständen von jeweils 5 Min wird 1 ml Lysis-Mischung (1% Brij58, 0.4% Na-Deoxycholat, 60 mA EDTA, 50 mM Tris-HCl pH 8.0) zugegeben. Nach 5–10 Min Lysis bei Raumtemperatur wird für 45 Min zentrifugiert (Sorvall, SS34 Rotor, 17 000 UpM) und der Überstand in ein neues Schraubkappenglas überführt. Nach Zugabe von 1 mol 3M NaClO$_4$ und 4 ml Dichlormethan wird kräftig geschüttelt und zur Phasentrennung in einer Tischzentrifuge 2 Min bei 3000 UpM zentrifugiert. Die obere wässrige, DNShaltige Phase wird abgehoben und in ein neues Schraubkappenglas überführt.

Die wässrige Phase wird so oft mit Dichlormethan ausgeschüttelt, bis keine Proteine mehr freigesetzt werden. Die Proteine banden zwischen der unteren Dichlormethanphase und der obrigen wässrigen Phase.

Der proteinfreien wässrigen Phase werden dann pro ml 0.54 ml Isopropanol tropfenweise zugegeben und 5 Min in der Sorvall, 15 000 upM zentrifugiert (SS34 Rotor). Der Niederschlag wird getrocknet und in 50 µl doppelt destilliert H$_2$O resuspendiert.

Zur schnellen Analyse werden Plasmide nach der Methode von Holmes und Quigley präpariert (D.S. Holmes und M. Quigley (1981), Analyt. Biochem. 114, 193).

1.5 ml einer Übernachtkultur in LB-amp (s.o.) werden 2 Min in einer Tischzentrifuge zentrifugiert, der Niederschlag in 0.4 ml STET-Puffer (8% Saccharose, 0.5% Triton X100, 50 mM EDTA, 50 mM Tris-HCl pH 8.0) resuspendiert und nach Zugabe von 25 µl Lysozym (10 mg/ml in 10 ml Tris-HCl pH 8.0) 30 Sek gekocht.

Nach 10 Min Zentrifugation wird der schleimige Niederschlag mit einem Zahnstocher entfernt, 1/10 Volumen 3 M Na-Acetat pH 7.5 und das gleiche Volumen Isopropanol zugegeben. Die Mischung wird 10 Min bei −20°C aufbewahrt, anschließend 5 Min in einer Tischzentrifuge zentrifugiert. Der Niederschlag wird dann mit 1 ml 70% Äthanol überschichtet und nochmal 3 Min zentrifugiert. Der Niederschlag wird getrocknet und in 50 µl doppelt destilliertem H$_2$O resuspendiert.

Beispiel 2

Transformation von E. coli K 12 Zellen, (modifiziert nach Cohen et al (1972), Proc. Natl. Acad. Sci. (69, 21110.)

Eine Übernachtkultur zu transformierender Zellen in LB Medium (10 g Trypton, 5 g Yeast Extract, 5 g Nael pro l doppelt destilliertem H$_2$O) wird 1/100 in frisches LB Medium verdünnt und bei 30°C zu einer OD$_{600}$ von 0.5 bis 0.8 schüttelnd inkubiert. Die Zellen werden zentrifugiert (Sorvall, SS34 Rotor, 5 Min 10 000 UpM) und in 13 ml eiskaltem 50 mM CaCl$_2$ resuspendiert. Nach nochmaliger Zentrifugation werden die Zellen in 1.3 ml eiskaltem CaCl$_2$ resuspendiert und 30 Min auf Eis gestellt. Zu 0.2 ml solcher kompetenter Zellen werden 1–10 µl zu transformierender DNS, z.B. aus einem Ligaseansatz hinzugegeben. Nach 30 Min Inkubation auf Eis werden die Zellen 3 Min bei 42°C und 1 Min auf Eis gehalten. Nach Zugabe von 2 ml LB Medium werden die Zellen 60 Min bei 30°C inkubiert, danach auf Ampicillin-haltige YT-Platten (5 g NaCl, 8 g Tryptone, 5 g Yeast Extrakt, 15 g Agar, 100 µg/ml Ampicillin, ggf. mit 20 µg/ml x-gal als Farbmarker und 10$^{-3}$M IPTG als lac-Induktor (U.

Rüther et al. (1981), Nucl. Acids Res. 9 4087) ausgestrichen und bei 30°C oder 37°C bebrütet.

Beispiel 3

Präparation von pMM5

1 µg des Plasmids pUR250 (U. Rüther et al (1982), Nucl. Acids Res. 10, 5765) werden in 30 µl HindIII-Puffer (50 mM NaCl, 10 mM Tris-HCl-pH 7,5, 10 mM mgCl$_2$, 1 mM DTT) mit 2 U PvuII für 2 Std bei 37°C inkubiert, 10 Min bei 65°C erhitzt und 5 µl zur Kontrolle der Restriktionsverdauung entnommen. Nach Überprüfung der erfolgten Restriktionsverdauung auf einem 0.7%igem Agarose-Gel wird 1.25 µl 1M NaCl und 2 U BamHI zugegeben und für weitere 2 Std bei 37°C inkubiert. Nach Überprüfung der BamHI-Restriktionsverdauung werden 5 µl dieses Reaktionsansatzes (ca. 150 ng) mit 5 µl (ca. 200 ng) PvuII/BamHI-gespaltener pBR322 DNS (F. Bolivar et al (1977), Gene 2, 95) gemischt und 10 Min bei 65°C erhitzt. Nach Zugabe von 2.5 µl 1 M Tris-HCl pH 7.5, 0.5 µl 1 M MgCl$_2$, 5ul 0.1 M DTT, 5 µl 10 mM ATP und 27 µl doppelt destilliertem H$_2$O (Ligase Puffer) sowie 1 µl T4-DNS-Ligase (1 U/µl) 16 Std bei 8°C ligiert. Die DNS wird nach der Ligierung 10 Min bei 65°C erhitzt und 5 µl dieser DNS für die Transformation (s. Beispiel 2) mt 0.2 ml kompetenter Zellen des E.coli K12 Stammes CSH52 (J. Miller (1972), Experiments in Molecular Genetics, Cold Spring Harbor Laboratory, New York) gemischt. Die transformierten Zellen werden auf YT-xgal-amp Platten ausgestrichen und die Platten 16 Std bei 37°C inkubiert. Von diesen Platten werden blaue Transformanten isoliert.

CSH52 ist ein lac-Wild-Typ Stamm. Die lac-Repressormoleküle dieses Stammes werden durch die in pBR322 klonierte lac-Operator-DNS auf dem PvuII/BamHI Fragment von pUR250 gebunden, was zur Induktion des lac-Operons führt. Dadurch wird β-Galaktosidase gebildet, die den farblosen x-gal Farbstoff in den YT-xgal-amp Platten zu einem blauen Indigo-Derivat abbaut.

Aus blauen Kolonien werden Plasmide präpariert und ein Plasmid (pMM5) von ca. 2860 bp mit zwei HindII-Restriktionsschnittstellen isoliert.

Beispiel 4

Präparation von pMM6

1 µg pMM5 DNS wird in 30 µl HindIII-Puffer (s. Beispiel 3) mit 2 U PvuII gespalten, 5 µl (ca. 150 ng) dieser DNS mit 1 µl phosphoryliertem BglII-Linker (1 µg/2 µl) in 50 µl Gesamtvolumen bei Raumtemperatur mit 3 U T4-DNS-Ligase in Ligase Puffer (s. Beispiel 3) 16 Std bei 8°C inkubiert. Danach wird das Volumen auf 300 µl mit doppelt destilliertem H$_2$O aufgefüllt und mit Chloroform extrahiert. Hierzu werden zu den 300 µl 200 µl Chloroform/Isoamylalkohol (24:1) hinzugegeben, geschüttelt und zur Phasentrennung kurz zentrifugiert. Die wässrige Phase wird in ein frisches Probengefäß überführt, 3mal mit 1 ml Äther extrahiert und mit Äthanol die DNS gefällt. Hierzu wird nach Zugabe von 30 µl 3m Na-Acetat pH 5.0 und 0.8 ml Äthanol die DNS 16 Std bei −20°C inkubiert, dann 15 Min bei 4°C mit 17 000 UpM in der Sorvall zentrifugiert. Der Überstand wird verworfen, der Niederschlag mit 1 ml 70% Äthanol überschichtet und nochmal 5 Min in einer Zentrifuge zentrifugiert. Der Überstand wird abermals verworfen, der Niederschlag getrocknet und in 27 µl doppelt destilliertem $H_2O$ resuspendiert. Nach Zugabe von 3 µl HindIII-Puffer (s. Beispiel 3) und 2 U PvuII wird die DNS für 2 Std bei 37°C inkubiert, anschließend 10 Min bei 65°C erhitzt. 10 µl der DNS wird in kompetente Zellen des E.coli K12 Stammes CSH52 (s. Beispiel 2) transformiert und Transformanten nach 16 Std Inkubation auf YT-amp Platten isoliert. Aus den Transformanten werden Plasmide präpariert (s. Beispiel 1) und ein PvuII-resistentes, BglII linearisierbares Plasmid (pMM6) isoliert.

Beispiel 5
Präparation vom pMM7

500 ng pMM6 DNS werden in 20 µl HindIII-Puffer (s. Beispiel 3) mit 5 U EcoRI 1 Std bei 37°C inkubiert, 10 Min bei 65°C erhitzt und nach Zugabe von 1 µl 2 mM dTTP, 1 µl 2 mM dATP und 1 µl DNS Polymerase I (0.4 U/µl) 15 Min bei Raumtemperatur inkubiert. Die DNS wird 10 Min bei 65°C erhitzt und 10 µl der DNS in 50 γl Ligase-Puffer (s. Beispiel 3) 16 Std bei 8°C mit 1 U T4-Ligase ligiert. Danach wird eine Chloroformextraktion und Äthanolfällung durchgeführt (s. Beispiel 4). Die DNS wird in 27 µl doppelt destilliertem $H_2O$ resuspendiert und nach Zugabe von 3 µl 10xEcoRI-Puffer (1 M NaCl, 500 mM Tris-HCl pH 7,5, 100 mM $M_gCl_2$, 10 mM DTT) mit 5 U EcoRI 2 Std bei 37°C inkubiert. 10 µl dieser DNS wird in den kompetenten E.coli K12 Stamm MM82-1: (lac-pro)$_\Delta$ thi ara strA r$^-$m$^+$ recA val$^R$ (Ø80dlacZ$^-$M15) transformiert. Aus auf YT-amp Platten gewachsenen Transformanten werden Plasmide präpariert (s. Beispiel 1) und ein EcoRI-resistentes Plasmid (pMM7) isoliert.

Beispiel 6
Präparation von pMM8

1 µg pMM7 DNS wird in 30 µl ClaI-Puffer (10 mM Tris-HCl pH 7.5, 10 mM $MgCl_2$, 1mM DTT) mit 2 U ClaI für 2 Std bei 37°C inkubiert, 10 Min bei 65°C erhitzt und 5 µl zur Analyse auf einem 0.7% Agarose-Gel entnommen. Nach Zugabe von 2.5 µl 1M NaCl und 2 U BamHI wird weitere 2 Std bei 37°C inkubiert, anschließend für 10 Min bei 65°C erhitzt. 1 µg pUK230 (M. Koenen et al. (1982), EMBO J. 1, 509) wird in 30 µl ClaI-Puffer (s.o.) mit 2 U HpaII für 2 Std bei 37°C inkubiert, 5 µl zur Kontrolle entnommen und 10 Min bei 65°C erhitzt.

Nach Zugabe von 2.5 µl 1 M NaCl und 2 U BamHI wird für weitere 2 Std bei 37°C inkubiert und die Reaktion durch 10-minütiges Erhitzen bei 65°C gestoppt.

Je 5 µl ClaI/BamHI verdaute pMM7 DNS und HpaII/BamHI verdaute pUK230 DNS werden gemischt und in 40 µl Ligase-Puffer mit 1 U T4-Ligase für 16 Std bei 8°C ligiert. Die DNS wird für 10 Min bei 65°C erhitzt und in kompetente Zellen des E.coli K12 Stammes MM 82-1 (s. Beispiel 5) transformiert. Auf YT-amp-xgal-IPTG-Platten werden blaue Transformanten isoliert. Blaue Transformanten entstehen durch alpha Komplementation (K.E. Langley et al. (1975), Proc. Acad. Sci. 72, 1254) zwischen der defekten chromosomal kodierten β-Galaktosidase und der Plasmid-kodierten lac-alpha-Region.

Beispiel 7
Präparation von pEM1

Das Plasmid pBR322-lac22 (M. Mieschendahl et al. (1981), Mol. Gen. Genet 183, 202) trägt die λ-Immunitätsregion bis zum 22. Aminosäurecodon des λ-N Gens. Das Repressor-Gen auf diesem Plasmid trägt die Temperaturmutation cI857.

200 ng pBR322-lac22 werden mit 2 U BglII in 20 µl BglII Puffer (s. Beispiel 8) 2 Std bei 37°C inkubiert. 10 Min bei 65°C erhitzt und 10 µl dieser DNS in 50 µl Ligase-Puffer mit 1 U T4-DNS-Ligase 16 Std ligiert. Die Reaktion wird durch 10-minütiges Erhitzen auf 65°C gestoppt, 10 µl dieser DNS in kompetente Zellen des E.coli K12 Stammes CSH52 (s. Beispiel 3) transformiert und auf YT-amp-Platten bei 30°C inkubiert.

Das Plasmide mit dereprimiertem λ-P$_1$ Promotor oft instabil sind (M. Rosenberg et al. (1983), Methods in Enzymology 101, p 123), können neben dem Ausgangsplasmid und falschen in vivo Rekombinanten nur solche, gesuchte Plasmide überleben, die das BglII DNS-Fragment mit dem λ-Repressor-Gen nicht verloren haben und dadurch den λ-P$_1$-Promotor reprimieren können.

Beispiel 8
Präparation von pRM1

1 µg pEM1 wird in 30 µl BglII Puffer (10 mM Tris-HCl pH 7.5, 10 mM MgCl$_2$, 1 mM DTT) mit 2 U BglII für 2 Std bei 37°C inkubiert, 10 Min bei 65°C erhitzt und nach Entnahme von 5 µl zur Kontrolle 2.5 µl 1 M NaCl zugegeben. Die DNS wird dann mit 2 U AluI für 2 Std bei 37°C inkubiert und die Reaktion durch 10-minütiges Erhitzen auf 65°C abgestoppt.

1 µl pMM8 wird in 30 µl HindIII-Puffer (s. Beispiel 3) mit 2 U BamHI 2 Std bei 37°C inkubiert und 5 µl zur Analyse der Restriktionsverdauung auf einem 0.7% Agarose-Gel entnommen. Zur Inaktivierung von BamHI wird die DNS 10 Min bei 65°C inkubiert. Nach Zugabe von 2.5 µl einer Lösung mit je 100 µM dATP, dGTP, dCTP und dTTP und 1 µl DNS-Polymerase I (0.4 U/µl) zum Auffüllen der BamHI 5'– überstehenden Enden wird 15 Min bei Raumtemperatur inkubiert. Die DNS wird mittels Chloroformextraktion und Äthanolfällung (s. Beispiel 4) gereinigt und in 30 µl BglII-Puffer (s.o.) resuspendiert. Nach Zugabe von 2 U BglII wird die DNS für 2 Std bei 37°C und für 10 Min bei 65°C inkubiert.

5 µl dieser DNS werden mit 5 µ BglII/AluI gespaltener pEM1 DNS gemischt und mit 3 U T4-DNS-Ligase in Ligase-Puffer (s. Beispiel 3) 16 Std bei 8°C inkubiert. Die DNS wird dann 10 Min bei 65°C erhitzt und 10 µl dieser DNS mit 8 µl doppelt destilliertem $H_2O$ und 2 µl 10xHindIII-Puffer (500 mM NaCl, 100 mM Tris-HCl pH 7,5, 100 mM MgCl$_2$, 10 mM DTT) gemischt. Nach Zugabe von 2 U HindIII wird für 2 Std bei 37°C, anschließend für 10 Min bei 65 °C inkubiert. Die DNS wird in kompetente Zellen des E.coli K12 Stammes MM82-1 (λ cl857S7) transformiert[1] und bei 30 °C auf YT-amp Platten Transformanten isoliert. Durch die HindIII Restriktionsverdauung im Anschluß an die Ligierung werden beide Ausgangsplasmide, pEM1 und pMM8, gespalten, so daß vornehmlich rekombinante Plasmide transformiert werden sollten. Ein λ-lysogener Stamm wird transformiert, da Plasmide mit dereprimiertem $P_1$-Promotor nicht lebensfähig sind (s. Beispiel 7).

Beispiel 9
Präparation von pRM9

1 µg pUR222 (U. Rüther et al. (1981), Nucl. Acids Res. 9, 4087) wird mit 5 U EcoRI in 30 µl HindIII-Puffer (s. Beispiel 3) für 2 Std bei 37 °C inkubiert, nach Entnahme von 5 µl zur Kontrolle für 10 Min bei 65 °C erhitzt. Nach Zugabe von 3 U AluI wird die DNS nochmals für 2 Std bei 37 °C und für 10 Min bei 65 °C inkubiert.

1 µg pRM1 wird mit 2 U BamHI in 30 µl BamHI-Puffer (100 mM NaCl, 10 mM Tris-HCl pH 7.5, 5 mM $MgCl_2$, 1 mM DTT) 2 Std bei 37 °C inkubiert, 5 µl zur Analyse auf einem 0.7% Agarose-Gel entnommen und die DNS 10 Min bei 65 °C erhitzt. Die DNs wird mit Chloroform extrahiert, mit Äthanol gefällt (s. Beispiel 4) und in 100 µl S1-Puffer (200 nM NaCl, 30 mM Na-Acetat pH 4.5, 5 mM $ZnSO_4$) mit 200 U S1 30 Min bei Raumtemperatur inkubiert.

Die DNS wird wiederum mit Chloroform extrahiert, mit Äthanol gefällt und in 30 µl HindIII-Puffer (s. Beispiel 3), 100 µM dGTP mit 0.4 U DNS-Polymerase I 15 bei Raumtemperatur inkubiert. Die Reaktion wird durch Erhitzen für 10 Min bei 65 °C gestoppt und die DNS nach Zugabe von 5 U EcoRI 2 Std bei 37 °C inkubiert.

200 ng dieser so behandelten pRM1 DNS wird nach 10-minütigem Erhitzen bei 65 °C mit 200 ng AluI/EcoRI gespaltener pUR222 DNS in 50 µl Ligase-Puffer (s. Beispiel 3) mit 2 U T4-DNS-Ligase für 16 Std bei 9 °C ligiert. Die Reaktion wird durch 10-minütiges Erhitzen bei 65 °C abgestoppt und in kompetente Zellen des E-coli K12 Stammes MM82-1 (λ cl857S7) transformiert.[2] Je 100 Transformanten, die auf YT-amp Platten bei 30 °C gewachsen waren, werden auf zwei YT-xgal-IPTGamp Platten ausgestrichen und bei 30 °C inkubiert. Nach 5 Std wird eine der Platten bei 37 °C, die andere Platte bei 30 °C für 11 Std weiter inkubiert. Es werden Transformanten isoliert, die bei 30 °C weiße Kolonien auf YT-xgal-amp Platten bilden, auf den gleichen Platten bei 37 °C jedoch nur einen blauen Hintergrund zeigen.

[1] Die transformierten Zellen wurden am 16. Mai 1984 bei der Deutschen Sammlung von Mikroorganismen hinterlegt; DSM 2690.

[2] Die transformierten Zellen wurden am 15. Mai 1984 bei der Deutschen Sammlung von Mikroorganismen hinterlegt; DSM 2961.

Beispiel 10
Konstruktion von pUM II-T17

1 µg pUMI-T17 DNS (M. Mieschendahl (1981), Dissertation Universität Köln) werden in 20 µl HindIII-Puffer mit 2 U EcoRI 2 Std bei 37 °C inkubiert, 10 Min bei 65 °C erhitzt und nach Zugabe von 3 µl 1M NaCl und 6 µl doppelt destilliertem $H_2O$ mit 2 U SalI weitere 2 Std bei 37 °C inkubiert.

1 µg pUMII DNS wird in 20 µl HindIII-Puffer mit 2 U EcoRI 2 Std bei 37 °C inkubiert, anschließend 10 Min bei 65 °C erhitzt. Das Plasmid pUMII ist ein Derivat des Plasmids pUMI, bei dem das den lac-Promotor/Operator-tragende PstI/EcoRI-Fragment von pUMI durch das kleinere PstI/EcoRI-Fragment von pBR322 ersetzt ist.

200 ng EcoRII/SalI verdauter pUMI-T17 DNS wird mit 200 ng EcoRI-gespaltener pUMII DNS in 50 µl Ligase-Puffer mit 1 U T4-DNS-Ligase 16 Std bei 8 °C ligiert, 10 Min bei 65 °C erhitzt und in kompetente Zellen des E.coli K12 Stammes DP54-1: (lac pro) thi recA rif[r]str A (M. Mieschendahl (1981), Dissertation Universität Köln) transformiert. Auf YT-xgal-amp Platten werden blaue Transformanten isoliert.

Beispiel 11
Konstruktion von pTP1 bis pTP5
a) Verkürzung von BglII-gespaltenem pUMII-T17 mit Bal31 und Einbau einer Bam-HI-site.

Plasmid pUMII-T17 wird in dem E.coli-Stamm CSH52 nach der Methode von Clewell und Helinski (1969) (Proc. Natl. Acad. Sci. 62: 1159–1166) vermehrt, isoliert und gereinigt. 50 µg gereinigte pUMII-T17 DNS wird mit 25 U BglII in 250 µl BglII-Puffer (s. Beispiel 8) für 4 Std bei 37 °C inkubiert. Die Reaktion wird durch 10 Min Erwärmen auf 65 °C gestoppt und die DNS durch Chloroformextraktion gereinigt, mit Alkohol gefällt und getrocknet, (s. Beispiel 4). Die DNS wird in 500 µl Bal31-Puffer (12 mM $CaCl_2$, 12 mM $MgCl_2$, 600 mM NaCl, 1mM EDTA, 10 mM Tris/HCl, pH 8.1) gelöst und mit 5 U Bal31 (Bethesda Research Laboratories) bei 30 °C inkubiert. Nach 2, 3, und 4 Min werden Proben à 160 µl entnommen und die Reaktion durch Pipettieren in 160 µl 10 mM Tris/HCl pH 8.0, 25 mM EDTA und Erwärmen auf 65 °C für 5 Min gestoppt. Die DNS wird durch Extraktion mit Chloroform gereinigt, mit Alkohol gefällt und getrocknet.

Um das Ausmaß der Verkürzung von pUMII-T17 durch Bal31 abzuschätzen, werden die 2, 3 und 4 Min Bal31 behandelten DNS-Aliquots in jeweils 20 µl HindIII-Puffer (s. Beispiel 3) gelöst und davon 2.5 µl (2 µg) mit 3 U HindIII in 20 µl HindIII-Puffer für 2 Std bei 37 °C inkubiert. Die entstandenen Fragmente werden auf einem 1% Agarose-Gel zusammen mit AluI gespaltenem pBR322-Markerfragmenten elektrophoretisch aufgetrennt.

Ca. 4 µg der mit Bal31 behandelten pUMII-T17 DNS werden mit 0.5 U Klenow-Polymerase und je 0.1 mM dGTP, dATP, DTTP und dCTP in 20 µl HindIII-Puffer für 20 Min bei Raumtemperatur behandelt und für 10 Min bei 65 °C inkubiert. Das Reaktionsgemisch wird mit Ligase-Puffer (s. Beispiel 3) auf ein Volumen von 50 µl gebracht und mit 500 ng phosphoryliertem BamEI-Linker (Biolabs, Sequenz: CGGATCCG) und 3 U $T_4$ DNS-Ligase bei 12°C für 16 Std ligiert. Die Ligasereaktion wird durch Erwärmen auf 65°C für 10 Min gestoppt, die DNS durch Extraktion mit Chloroform gereinigt und mit Alkohol gefällt. Die DNS wird in 50 µl BamHI-Puffer (s. Beispiel 9) gelöst, mit 5 U BamHI bei 37°C für 2 Std inkubiert, 10 Min auf 65°C erwärmt und in einem Volumen von 200 µl Ligasepuffer erneut mit 3 U $T_4$-DNS-Ligase 16 Std bei 12°C ligiert. Mit diesem Ligaseansatz werden kompetente Zellen des Stammes DP54-1 (s. Beispiel 10) wie in Beispiel 2 beschrieben transformiert und der Transformationssatz auf 2 YT-amp-xgal-Platten (s. Beispiel 2) ausgetrichen. Nach 18 Std Inkubation bei 37°C werden ca. 1000 blaue und etwa 25 weiße Kolonien erhalten. Von den weißen Kolonien wird nach der Methode von Holmes und Quigley (s. Beispiel 1) die Plasmid DNS isoliert. Je 300 ng der Plasmid DNS wird mit U BamHI in 20 µl BamHI-Puffer für 2 Std bei 37°C inkubiert und die DNS auf einem 1% Agarose-Gel durch Elektrophorese aufgetrennt. Nach der Trennung wird die DNS im Gel mit Ethidiumbromid gefärbt. Von etwa 250 Plasmiden zeigen sich etwa 90 durch BamHI linearisierbar. Von diesen 87 haben nach BamHI-Spaltung etwa 30 die richtige Größe von ca. 9.500 bp.

b) Isolierung des trpPO-Fragments aus pdR11

Plasmid pDR11 (D.R. Russel und G.N. Bennet (1982), Gene 17: 9–18), wird durch die Isopropanol-Methode (s. Beispiel 1) isoliert und gereinigt. 10 µg pDR11 werden mit 10 U AluI und 5 µg RNase in 50 µl HindIII-Puffer für 2 Std bei 37°C inkubiert und die entstandenen Fragmente durch Elektrophorese auf einem Polyacrylamid-Gel mit 7,5% Acrylamid, 0.258% Bis-acrylamid in 90 mM Tris, 90 mM Borsäure, 2.5 mM EDTA zusammen mit Markerfragmenten von AluI-gespaltenen pBR322 aufgetrennt (Maniatis et al., Molecular Cloning, Cold Spring Harbor Laboratory, 1982). Nach der Trennung wird die DNS im Gel mit Ethidiumbromid gefärbt und das Gelstück mit dem 41 bp trpPO-Fragment ausgeschnitten. Dieses Gelstück wird durch Drücken durch eine 1 ml Tuberkulinspritze zerkleinert und mit 1 ml Puffer (150 mM NaCl, 10 M Tris/HCl pH 0.8, 1 mM EDTA) für 16 Std bei 37°C geschüttelt. Das Eluat wird durch sterile Baumwollwatte filtriert und die DNS im Filtrat mit 5 µg Hefe t-RNS (Boehringer), 0,3 M Natriumacetat und 2,5 ml Alkohol bei −20°C gefällt, abzentrifugiert (20 000 UpM 10 Min, SW 50.1 Rotor der Firma Beckman) und getrocknet. Die DNS wird in 10 µl Ligase-Puffer gelöst und mit 100 ng phosphoryliertem BamHI-Linker-Fragment durch 2 U $T_4$-DNS-Ligase für 16 Std bei 12°C ligiert. Die Ligasereaktion wird durch Erwärmen für 10 Min auf 65°C gestoppt, die DNS durch Chloroformextraktion gereinigt, mit Alkohol gefällt und getrocknet. Die DNS wird in 30 µl BamHI-Puffer gelöst, mit 5 U BamHI bei 37°C inkubiert und für 10 Min auf 65°C erwärmt.

c) Zusammenbau und Test von pTP1 bis pTP5

Jeweils 0.5 µg Plasmid DNS der in Beispiel 11 erhaltenen Plasmide wird mit 1 U BamHI in 10 µl BamH1-Puffer für 2 Std bei 37°C gespalten, für 10 Min auf 65°C erwärmt. Die Plasmid DNS wird mit je 1 µl, mit BamHI-Linker ligierten, BamHI gespaltenen trpPO-Fragment aus Beispiel 11 b in 30 µl Ligasepuffer und je 1 U $T_4$-DNS-Ligase für 16 Std bei 12°C ligiert. Mit diesen Reaktionsansätzen werden jeweils kompetente Zellen des Stammes 82-1 (s. B. 5) lysogen für den Phagen cl857 $S_7$ (Hershey, A. Ed., The Bacteriophage, Cold Spring Harbor Laboratory, 1971) transformiert. Die Transformationsansätze werden anschließend in je 1.5 ml YT 2 Std bei 30°C geschüttelt, die Zellen mit physiologischer Kochsalzlösung gewaschen und auf 1 × A Minimal-Glucose-amp x-gal-Platten (J. Miller (1972), s.B. 1) ausgetrichen. Nach 48 Std Inkubation bei 42°C werden in 5 von etwa 27 Ansätzen blaue Kolonien auf den Platten erhalten.[1] Von jeweils einer Kolonie dieser 5 Ansätze wird nach der Methode von Holmes und Quigley Plasmid DNS präpariert und mit je ca. 10 ng dieser DNS kompetente Zellen des Stammes DP54-1 transformiert.

Die Transformationsansätze werden je zur Hälfte auf 1 × A Minimal-Glucose-amp-prolin-xgal-Platte ohne Tryptophan bzw. auf den gleichen Platten mit zusätzlich 100 µg/ml Tryptophan ausgestrichen. Nach 48 Std Inkubation wachsen auf den Platten ohne Tryptophan blaue Kolonien und auf den Platten mit Tryptophan weiße Kolonien. Die aus 5 verschiedenen Ansätzen erhaltenen Plasmide werden pTP1 bis pTP5 genannt.

Beispiel 12
Konstruktion von pEM11

0.5 µg pTP1 DNS werden in 20 µl HindIII-Puffer mit 1 U PvuII 2 Std bei 37°C inkubiert, danach 10 Min bei 65°C erhitzt. 100 ng dieser DNS wird mit 1 U $T_4$ DNS-Ligase 16 Std bei 8°C in 30 µl Ligase-Puffer ligiert, 10 Min bei 65°C erhitzt und die DNS in kompetente Zellen des E-coli K12 Stammes MM 82-1 transformiert (s. Beispiel 5 und 2). Es wird ein Derivat von pTP1 isoliert, bei dem drei PvuII-Fragmente deletiert sind.

Beispiel 13
Konstruktion von F′EM11 und Einbau ins Chromosom

Das Plasmid pEM11 wird in kompetente Zellen des Stammes E.coli K12 DP90CNal/F′lac 22A4 cl[+] transformiert.[2] Das Episom F′lac 22A4 cl[+] trägt eine λ N-lacZ[+] Genfusion mit einem cl[+] Allel (M. Mieschendahl et al., (1981), Molec. Gen. Genet. 183, 202). Zur Homogenoten-Bildung werden die Zellen fünfmal in $10^4$er Verdünnungsschritten bis zum Erreichen der stationären Phase in YT-Medium bei 37°C inkubiert (J. Miller (1972), Experiments in Molecular Genetics, Cold Spring Harbor

[1] Eine Kultur des pTP1-Stammes wurde am 15. Mai 1984 bei der Deutschen Sammlung von Mikroorganismen hinterlegt und hat die Nummer DSM 2962 erhalten.

[2] Hinterlegt am 15. Mai 1984 bei der Deutschen Sammlung von Mikroorganismen; Nr. DSM 2963.

Laboratory, New York). Danach werden 0.1 ml Zellsuspension auf eine 1 × A-Minimal-Lactose-tryptophan-Platte (J. Miller (1972) s.o.) ausgestrichen und nach 48 Std bei 37 °C lac⁺ Revertanten isoliert. Aus diesen lac⁺ Kolonien werden die Episome in den Stamm DP54-1 gekreuzt. Durch Ausstreichen eines F'EM11-Stammes auf Acridinorange haltigen Platten wird das Episom in Chromosom integriert (J. Miller (1972) s.o.)

## Patentansprüche

1. Verfahren zur Steuerung der Expression von in einem Vektor inserierten Genen in prokaryotischen Mikroorganismen durch Verwendung eines Vektors, der einen Expressionskomplex enthält, wobei der den Vektor enthaltende Mikroorganismus in einem Chromosom oder in einem mit dem Vektor verträglichen Plasmid oder Episom ein Kontrollelement enthält und die Steuerung der Expression durch Änderung der Konzentration von Aminosäuren durchgeführt wird, dadurch gekennzeichnet, daß man einen Vektor verwendet, der als Expressionskomplex lambda-$P_LO_L$ – lacZ als reprimierbaren Promoter mt Transkriptionssignalen bzw. lambda-$P_LO_L$ – lacZ' als reprimierbaren Promoter mit Transkriptions- und Translationssignalen sowie ein durch Tryptophan reprimierbares Repressorgen $P_{trp}O_{trp}$-lambda-cl enthält, und zur Steuerung der Expression Tryptophan oder tryptophanhaltige Peptide oder Proteine zugegeben werden.

2. Verfahren nach Anspruch 1, dadurch gekennzeichnet, daß der Vektor einen Polylinker der lacZ-Sequenz enthält, insbesondere einen Polylinker aus pUR222 oder pUR250 oder Teile davon.

3. Verfahren nach Anspruch 1 oder 2, dadurch gekennzeichnet, daß der Polylinker mindestens ein funktionelles Gen, ein Genabschnitt oder eine für Proteine kodierende cDNS verwendet wird.

4. Verfahren nach mindestens einem der Ansprüche 1 bis 3, dadurch gekennzeichnet, daß als Mikroorganismus E. coli verwendet wird.

5. Vektor zur Durchführung des Verfahrens nach mindestens einem der Ansprüche 1 bis 4, enthaltend den Expressionskomplex lambda-$P_LO_L$ – lacZ oder lambda-$P_LO_L$ – lacZ', dadurch gekennzeichnet, daß der Expressionskomplex einen für den Einbau von funktionellen Genen, Genabschnitten oder cDNS geeigneten Polylinker in der lacZ- bzw. der lacZ'-Sequenz aufweist.

6. Vektor nach Anspruch 5, dadurch gekennzeichnet, daß in den Polylinker mindestens ein funktionelles Gen, ein Genabschnitt oder eine für Proteine kodierende cDNS inseriert ist.

7. Vektor nach einem der Ansprüche 5 oder 6, dadurch gekennzeichnet, daß er die linke Promoter-Operator-Region des Bakteriophagen Lambda, verbunden mit dem 5'-Bereich des beta-Galactosidase-Gens aus E. coli, sowie den Polylinker aus pUR222 oder pUR250 oder Teile davon in diesem Bereich enthält.

8. Plasmid oder Episom, dadurch gekennzeichnet, daß es das cl-Repressor-Gen des Bakteriophagen Lambda unter der Kontrolle der Promoter-Operator-Region des Tryptophan-Operons von E. coli enthält.

9. Prokaryotischer Mikroorganismus, enthaltend in seinem Chromosom das cl-Repressor-Gen des Bakteriophagen Lambda unter der Kontrolle der Promoter-Operator-Region des Tryptophan-Operons von E. coli.

10. Prokaryotischer Mikroorganismus, enthaltend ein das cl-Repressor-Gen des Bakteriophagen Lambda unter der Kontrolle der Promoter-Operator-Region des Tryptophan-Operons von E. coli enthaltendes Plasmid oder Episom.

## Claims

1. Method of controlling the expression of genes inserted in a vector in prokaryotic microorganisms by using a vector containing an expression complex, the microorganism containing the vector carrying a control element in a chromosome or in a plasmid or episome that is compatible with the vector, and the expression being controlled by changing the concentration of amino acids, comprising: using a vector which contains as expression complex lambda-$P_LO_L$ – lacZ as repressible promotor including the signals for transcription or lambda-$P_LO_L$ – lacZ' as repressible promotor including the signals for transcription and translation, as well as a repressible repressor gene $P_{trp}O_{trp}$-lambda-cl; and, in order to control the expression, adding tryptophan or tryptophan-containing peptides or proteins.

2. Method as claimed in claim 1, wherein the vector contains a polylinker of lacZ sequence, preferably a polylinker of pUR222 or pUR250 or parts thereof.

3. Method as claimed in claim 1 or claim 2, wherein the polylinker contains at least one functional gene, one gene segment or a cDNS coding for proteins.

4. Method as claimed in at least one of the claims 1 to 3, wherein E. coli is used as microorganism.

5. Vector for performing the method as claimed in at least one of the claims 1 to 4, containing the expression complex lambda-$P_LO_L$ – lacZ or lambda-$P_LO_L$ – lacZ', wherein the expression complex contains a polylinker which is suitable for insertion of functional genes, gene segments or cDNS in the lacZ or lacZ' sequence.

6. Vector as claimed in claim 5, wherein at least one functional gene, one gene segment or a cDNS coding for proteins is inserted in the polylinker.

7. Vector as claimed in claim 5 or claim 6, which contains the left promotor-operator region of the bacteriophage lambda, combined with the 5' region of the beta-galactosidase gene from E. coli, as well as the polylinker from pUR222 or PuR250 or parts thereof in said region.

8. Plasmid or episome which contains the cl repressor gene of the bacteriophage lambda controlled by the promotor-operator region of the tryptophan operon of E. coli.

9. Prokaryotic microorganism which contains in its chromosome the cl repressor gene of the bacteriophage lambda controlled by the promotor-operator region of the tryptophan operon of E. coli.

10. Prokaryotic microorganism which contains a plasmid or episome containing the cl repressor gene of the bacteriophage lambda controlled by the promotor-operator region of the tryptophan operon of E. coli.

**Revendications**

1. Procédé pour la régulation de l'expression, dans des micro-organismes procaryotes, de gènes insérés dans un vecteur, par utilisation d'un vecteur qui contient un complexe d'expression, le micro-organisme contenant le vecteur contenant un élément régulateur dans un chromosome ou dans un plasmide ou épisome compatible avec le vecteur, et la régulation de l'expression étant effectuée par modification de la concentration d'aminoacides, caractérisé en ce que l'on utilise un vecteur qui contient en tant que complexe d'expression lambda-$P_L O_L$-lacZ en tant que promoteur réprimable avec des signaux de transcription ou lambda-$P_L O_L$-lacZ' en tant que promoteur réprimable avec des signaux de transcription et de traduction, ainsi qu'un gène répresseur $P_{trp} O_{trp}$-lambda-cl réprimable par le tryptophane, et du tryptophane ou des peptides ou protéines contenant du tryptophane étant ajoutés pour la régulation de l'expression.

2. Procédé selon la revendication 1, caractérisé en ce que le vecteur contient un polylinker de la séquence lacZ, en particulier un polylinker provenant de pUR222 ou de pUR250 ou des fragments de celui-ci.

3. Procédé selon la revendication 1 ou 2, caractérisé en ce que l'on utilise pour le polylinker au moins un gène fonctionnel, un fragment de gène ou un ADN codant pour des protéines.

4. Procédé selon au moins l'une des revendications 1 à 3, caractérisé en ce que l'on utilise, en tant que micro-organisme, E. coli.

5. Vecteur pour la mise en œuvre du procédé selon au moins l'une des revendications, 1 à 4, contenant le complexe d'expression lambda-$P_L O_L$-lacZ ou lambda-$P_L O_L$-Z', caractérisé en ce que le complexe d'expression comporte dans la séquence lacZ ou la séquence lacZ' un polylinker approprié à l'incorporation de gènes fonctionnels, de fragments de gène ou d'ADNc.

6. Vecteur selon la revendication 5, caractérisé en ce que, dans le polylinker est inséré au moins un gène fonctionnel, un fragment de gène ou un ADN codant pour des protéines.

7. Vecteur selon l'une des revendications 5 ou 6, caractérisé en ce qu'il contient la région promoteur-opérateur gauche du bactériophage lambda, liée à la région (5') du gène de la β-galactosidase de E. coli, ainsi que le polylinker provenant de pUR222 ou pUR250 ou des fragments de celui-ci, dans cette région.

8. Plasmide ou épisome, caractérisé en ce qu'il contient le gène répresseur cl du bactériophage lambda sous le contrôle de la région opérateur-promoteur de l'opéron tryptophane de E. coli.

9. Micro-organisme procaryote contenant dans son chromosome le gène répresseur cl du bactériophage lambda sous le contrôle de la région opérateur-promoteur de l'opéron tryptophane de E. coli.

10. Micro-organisme procaryote contenant un plasmide ou épisome contenant le gène répresseur cl du bactériophage lambda sous le contrôle de la région opérateur-promoteur de l'opéron tryptophane de E. coli.

Fig.1                                                    Fortsetzung

Fig.1 Fortsetzung 1

Fortsetzung

Fig.1  Fortsetzung 2

EP 0 146 572 B1

Fig. 2

## ohne Trp

λPO

trp PO    λ cI

## mit Trp

λPO

trp PO    λ cI

Chromosom

| | |
|---|---|
| λcI | λ Repressor Gen |
| | λ Repressor |
| λPO | λ Kontrollregion |
| | Kloniertes Gen |
| • | Genprodukt |
| ◇ | Tryptophan Repressor |
| trp PO | Tryptophan Kontrollregion |

Fig. 3

19